Europäisches Patentamt

European Patent Office

Office européen des brevets

(19)

(11) Numéro de publication: **0 262 043 B1**

(12) **FASCICULE DE BREVET EUROPEEN**

(45) Date de publication de fascicule du brevet: **13.05.92**

(51) Int. Cl.5: **C12N 15/00**, C07H 21/04, C12N 5/00, C12N 7/00, C12N 15/39, C12N 15/86

(21) Numéro de dépôt: **87402104.1**

(22) Date de dépôt: **21.09.87**

(54) **Séquence d'ADN vecteurs, virus recombinants et procédé mettant en oeuvre des virus de la vaccine recombinants capables de se multiplier dans les cellules CHO.**

(30) Priorité: **23.09.86 FR 8613272**

(43) Date de publication de la demande:
**30.03.88 Bulletin 88/13**

(45) Mention de la délivrance du brevet:
**13.05.92 Bulletin 92/20**

(84) Etats contractants désignés:
**AT BE CH DE ES FR GB GR IT LI LU NL SE**

(56) Documents cités:
**EP-A- 0 110 385**
**EP-A- 0 162 782**

**EXPERIENTIA, vol. 38, no. 3, mars 1982, pages 285-297, Birkhäuser Verlag, Basel, CH; R. WITTEK: "Organization and expression of the poxvirus genome"**

**NUCLEIC ACIDS RESEARCH, vol. 10, no. 18, 1982, pages 5673-5679, IRL Press Ltd, Oxford, GB; B.M. BAROUDY et al.: "Sequence homologies of diverse length tandem repetitions**

**near ends of vaccinia virus genome suggest anequal crossing over"**

(73) Titulaire: **TRANSGENE S.A.**
**11, rue de Molsheim**
**F-67000 STRASBOURG(FR)**

(72) Inventeur: **Drillien, Robert**
**10, Boulevard Paul Déroulède**
**F-67000 Strasbourg(FR)**
Inventeur: **Spehner, Danièle**
**29, Rue de la Chênaie**
**F-67200 Eckbolsheim(FR)**

(74) Mandataire: **Warcoin, Jacques et al**
**Cabinet Régimbeau 26, avenue Kléber**
**F-75116 Paris(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 262 043 B1

# Description

Le virus de la vaccine est de plus en plus employé comme vecteur d'expression dans des cellules animales depuis la mise au point des méthodes propres à ce système (Panicali et Paoletti, 1982 ; Mackett et al., 1982 ; Smith et al., 1983 ; Panicali et al. ; Kieny et al., 1984). La construction de virus recombinants du virus de la vaccine contenant des gènes qui codent pour des protéines d'intérêt médical ou vétérinaire est particulièrement recherchée. La synthèse de la protéine étrangère dont le gène a été intégré dans le génome du virus de la vaccine peut ensuite être obtenue en culture cellulaire in vitro ou après inoculation à un organisme vivant, selon le but poursuivi. Un des avantages du virus de la vaccine et tant que vecteur est sa capacité de se multiplier dans un grand nombre de types cellulaires différents.

Cependant quelques exceptions à cette règle existent et, en particulier, le virus de la vaccine de type sauvage est incapable de se multiplier dans une lignée de cellules d'ovaires de hamster chinois, CHO (Drillien, Spehner et Kirn, 1978). Or les cellules CHO constituent un des systèmes les plus prometteurs pour la synthèse de protéines dans des cellules de mammifères. En effet ces cellules se cultivent facilement, elles ont un temps de génération court et leur génétique est la mieux maîtrisée de tous les systèmes analogues.

La présente invention concerne la modification du virus de la vaccine par l'intégration dans son génome d'un gène étranger qui lui confère la capacité de se multiplier dans les cellules CHO. Le gène apportant cette nouvelle spécificité d'hôte est dérivé du virus du cowpox (ou variole bovine, virus de la vache apparenté au virus de la vaccine) qui est capable de se multiplier dans les cellules CHO. Les génomes du cowpox et du virus de la vaccine sont très proches particulièrement dans les 100.000 paires de bases de la partie centrale de l'ADN (Mackett et Archard, 1979). Cependant le génome du cowpox est plus grand que celui de la vaccine (environ 230.000 pb au lieu de 190.000) et l'information génétique supplémentaire qu'il contient semble résider essentiellement dans ses extrémités. La similitude entre le génome du virus de la vaccine et celui du cowpox permet d'envisager d'employer le cowpox comme vecteur naturellement adapté aux cellules CHO.

Toutefois, le cowpox se multiplie à un titre dix fois inférieur à celui de la vaccine ce qui risque d'entraîner un rendement plus faible de l'expression d'une protéine produite par un recombinant cowpox en comparaison avec un recombinant vaccine.

D'autre part dans la perspective de l'utilisation de recombinants viraux pour la vaccination, il faut souligner que l'emploi du virus de la vaccine est bien maîtrisé et a permis à ce jour l'éradication complète la variole.

La présente invention concerne la mise au point d'un vecteur présentant les avantages connus du virus de la vaccine et la capacité de se multiplier sur cellules CHO du virus du cowpox.

La présente invention concerne, d'abord, l'identification et la localisation de l'information génétique qui confère au virus du cowpox la capacité de se multiplier dans les cellules CHO.

En effet, les études menées ont permis d'identifier une séquence impliquée dans la multiplication du virus cowpox dans les cellules CHO et qui, transférée dans le virus de la vaccine, assure la multiplication de ce virus dans les cellules CHO.

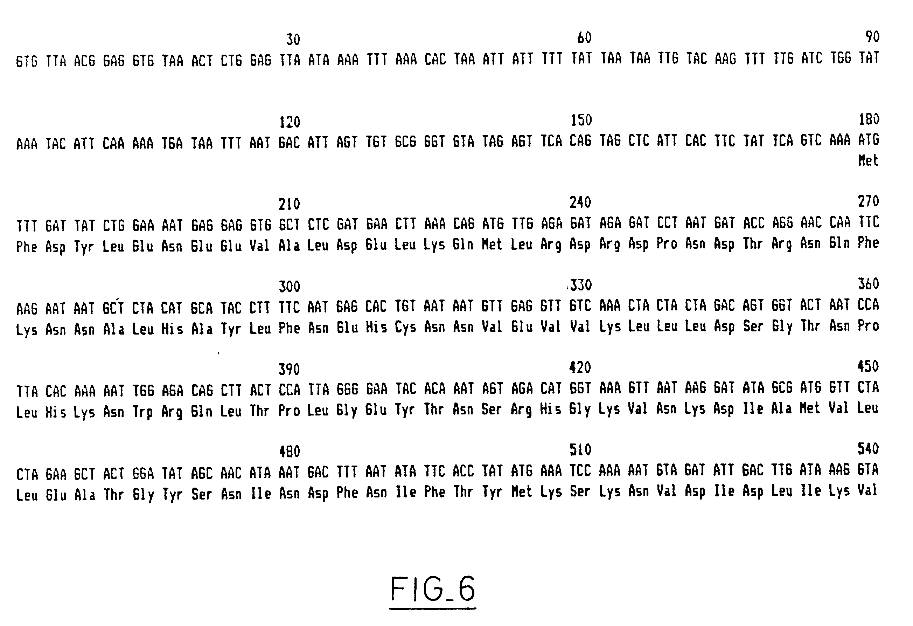

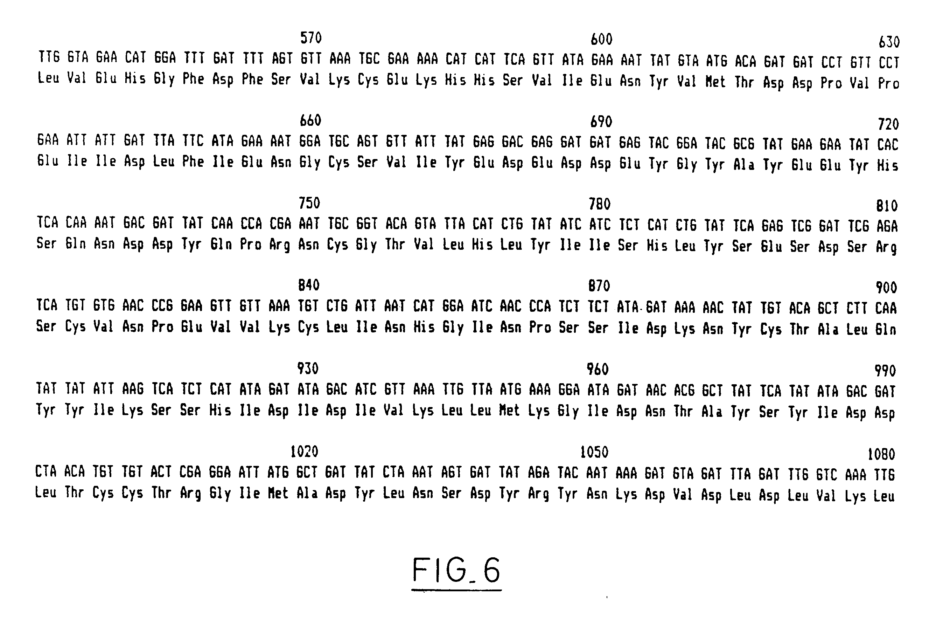

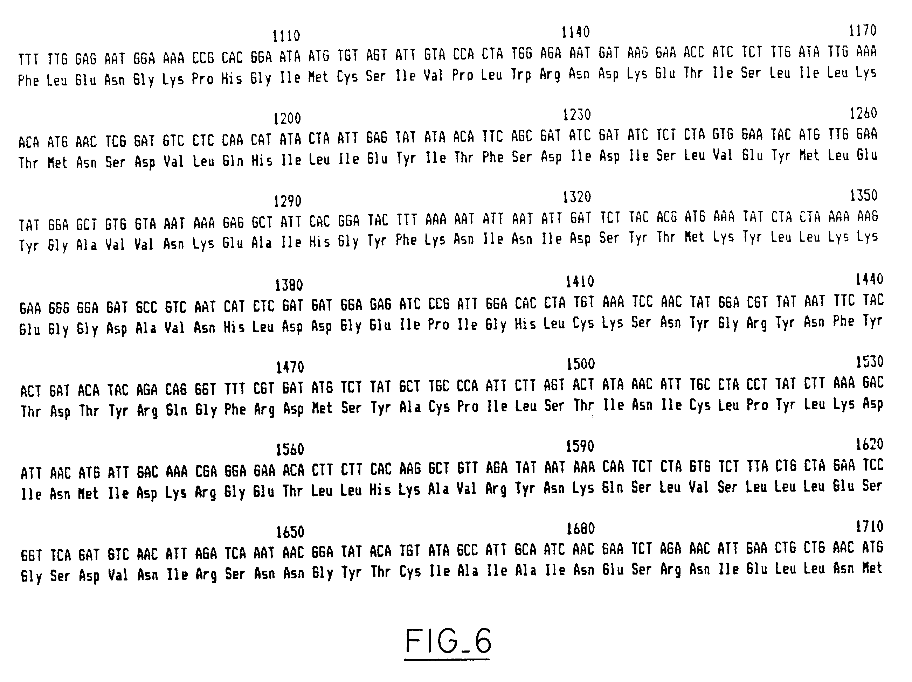

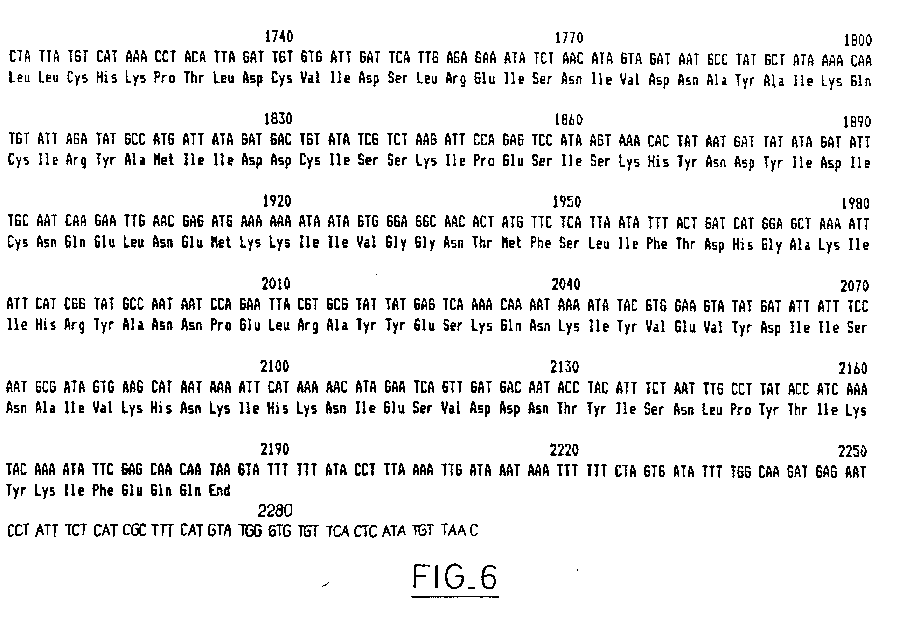

Ainsi, l'invention concerne une séquence d'ADN isolée notamment du virus du cowpox et qui participe à la multiplication de ce virus dans les cellules CHO et qui comprend la totalité ou une partie fonctionnelle de la séquence représentée dans la figure 6 ou une séquence équivalente fonctionnelle.

Il est possible qu'une partie de ce gène soit suffisante pour assurer la fonction de multiplication, on l'appelle "partie fonctionnelle". Il est également possible que des mutations ou des variations ponctuelles ne modifient pas la fonction, on parle alors de "séquence équivalente fonctionnelle".

Bien que l'on préfère que cette séquence d'ADN comporte ses propres signaux de contrôle qui assurent son expression dans les cellules CHO, ceci n'est pas indispensable et l'on peut envisager de la mettre sous le contrôle d'éléments ayant une autre origine.

Comme cela est décrit précédemment, cette séquence d'ADN est plus particulièrement destinée à être intégrée dans le virus de la vaccine pour assurer sa multiplication dans les cellules CHO. Cette intégration s'effectue par recombinaison homologue, il est donc intéressant de prévoir que la séquence d'ADN en cause comportera au moins une région homologue d'une séquence du virus de la vaccine qui pourra participer à ce processus de recombinaison homologue au cours de la multiplication intracellulaire des virus.

En fait, il apparaît que chez le virus cowpox le gène permettant la multiplication dans les cellules CHO est entouré de séquences homologues à des séquences du génome de la vaccine, ce qui permet de simplifier la préparation d'un vecteur plasmidique de recombinaison.

Comme cela est déjà connu pour le virus de la vaccine, il est possible d'insérer dans la séquence d'ADN, objet de l'invention, un gène codant pour une protéine d'intérêt industriel sous la dépendance d'éléments de contrôle assurant son expression dans les cellules hôtes. Cette technologie a déjà été décrite, notamment dans les brevets suivants : 84.06499, 84.07959, 85.09225 et peut être utilisée avec éventuellement certaines adaptations et avantages. En particulier, dans les contructions précédentes, la sélection des virus recombinants était effectuée en insérant le gène à exprimer dans le gène TK de la vaccine, ce qui a pour conséquence de rendre le virus recombinant TK⁻ et de permettre la sélection par le procédé connu.

Dans le cas présent, il n'est pas indispensable d'effectuer une insertion dans le gène TK car, dans la mesure où le gène codant pour la protéine d'intérêt industriel est lié au gène assurant la multiplication dans les cellules CHO, seuls les virus recombinants peuvent se multiplier sur cellules CHO, ce qui autorise une sélection "naturelle" des virus recombinants.

Dans ce dernier cas, il est préférable que le bloc d'ADN comprenant les gènes à recombiner soit flanqué de séquences homologues de séquences du virus de la vaccine afin qu'ils restent liés lors de la recombinaison.

La présente invention concerne également les cellules CHO infectées par un virus de la vaccine recombinant incorporant une séquence d'ADN telle que décrite précédemment et, en particulier, un gène codant pour une protéine d'intérêt industriel, ainsi que les virus correspondants.

L'invention concerne également les vecteurs plasmidiques incorporant une séquence d'ADN telle que décrite précédemment, ces vecteurs étant utilisables pour effectuer la recombinaison in vivo.

L'invention concerne également des cellules CHO ayant intégré une séquence d'ADN selon la présente invention et capables d'assurer la multiplication du virus de la vaccine dans ces cellules.

Enfin, l'invention concerne la préparation de protéines d'intérêt industriel par culture de cellules CHO infectées par un virus recombinant selon l'invention.

Les exemples ci-après sont destinés à illustrer d'autres caractéristiques et avantages de la présente invention.

Exemple 1 Identification de la région du génome du virus du cowpox qui permet la multiplication dans les cellules CHO.

Des recombinants entre le virus de la vaccine et le cowpox ont été sélectionnés après infection mixte de cellules d'embryons de poulet avec chacun des virus. L'analyse de l'ADN des recombinants montre que la capacité de se multiplier dans des cellules CHO est associée à la conservation des sites de restriction de l'extrémité gauche du génome du cowpox.

Des cellules primaires d'embryon de poulet, préparées à partir d'oeufs embryonnés de 11 à 12 jours, sont infectées simultanément avec un mutant thermosensible du virus vaccinal, tsN7 (Drillien et al., 1982) et le cowpox (souche Brighton) à raison de 2 unités formant plage (ufp) par cellule. Parallèlement d'autres tapis cellulaires sont infectés avec chacun de ces virus. Après une heure d'adsorption l'excés de virus non adsorbé est éliminé et du milieu frais est ajouté aux cellules.

Celles-ci sont incubées à 33°C pendant un à deux jours jusqu'à la nécrose totale de la couche cellulaire. Les cellules infectées sont alors congelées puis décongelées et le virus issu de l'infection est titré à 39,5°C sur cellules d'embryon de poulet, sous une couche de milieu contenant 1 % d'agar noble. Après deux jours à 39,5°C un plus grand nombre de plages de virus s'est formé sur les tapis cellulaires infectés avec le mélange des 2 virus que sur les tapis témoins (ni le mutant thermosensible du virus de la vaccine ni le cowpox ne donnent un nombre significatif de plages) ; les plages qui apparaissent à partir de l'infection mixte peuvent donc correspondre à des recombinants entre le cowpox et le virus de la vaccine.

Les plages de recombinants potentiels sont reprises individuellement et le virus qu'elles contiennent est amplifié par multiplication sur cellules d'embryon de poulet. Leur ADN est ensuite purifié, coupé par des enzymes de restriction puis analysé sur gel d'agarose.

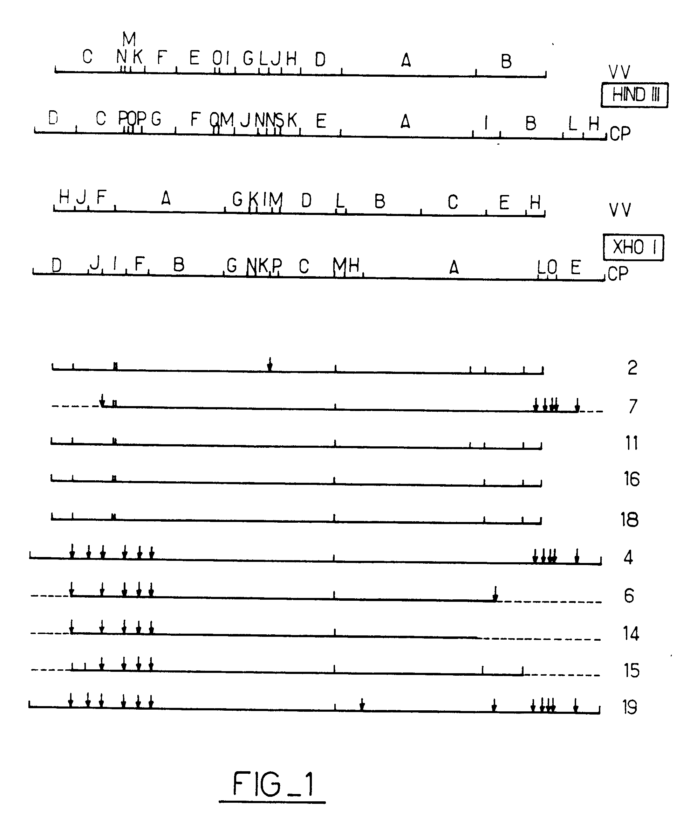

Les profils de restriction permettent de déduire que chaque plage correspond effectivement à un recombinant entre l'ADN du virus de la vaccine et l'ADN du cowpox. Grâce aux cartes de restriction connues pour les virus parentaux (Mackett and Archard 1978, Drillien and Spehner 1983) il est possible de reconnaître l'origine de la plupart des fragments des recombinants et de dresser leurs cartes de restriction (figure 1).

On voit que les recombinants dénommés 4, 6, 14, 15 et 19 qui sont capables de se multiplier dans des cellules CHO ont conservé les sites caractéristiques de l'extrémité gauche du génome du cowpox. Les autres recombinants dénommés 2, 7, 11, 16 et 18 qui sont incapables de se multiplier dans des cellules CHO ne possèdent que partiellement ou pas du tout ces sites de l'extrémité gauche du génome du cowpox.

Il ressort de ces résultats que la conservation des sites de restriction de l'extrémité gauche du génome du cowpox est associée au phénotype de multiplication sur cellules CHO.

Exemple 2 Isolement et analyse du génome de recombinants du virus de la vaccine ayant intégré un fragment d'ADN du cowpox.

Pour mieux préciser la localisation de l'information génétique utile, des recombinants capables de se multiplier sur cellules CHO ont été sélectionnés après infection avec le virus de la vaccine et transfection avec des fragments d'ADN du cowpox.

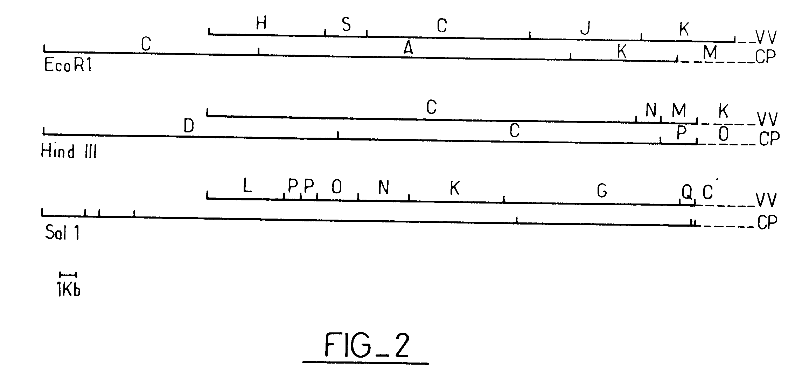

Les fragments de restriction utiles à l'analyse de la partie importante, c'est-à-dire l'extrémité gauche des 2 virus sont représentés dans la figure 2 ; c'est dans cette partie du génome que l'on peut soupçonner qu'interviennent les événements de recombinaison décrits dans l'exemple 1.

Des cellules primaires d'embryon de poulet sont infectées avec le mutant tsN7 du virus de la vaccine (Drillien et al., 1982) à raison de 0,1 ufp par cellule et transfectées avec un mélange d'ADN intact de la souche sauvage du virus de la vaccine (souche Copenhagen) et de l'ADN du cowpox (souche Brighton) préalablement digéré avec l'enzyme HindIII. Des témoins de transfection sand ADN ou avec seulement l'ADN du virus de la vaccine sont réalisés.

Après 48 heures d'incubation à 39.5°C les cellules sont congelées, décongelées puis le virus ainsi libéré est titré sur une monocouche de cellules CHO qui sont ensuite recouvertes de milieu contenant 1 % d'agar.

Les échantillons provenant de cellules transfectées avec l'ADN du cowpox donnent de nombreuses plages de lyse sur cellules CHO tandis que les échantillons témoins n'en donnent aucune.

Les plages visibles sur cellules CHO sont reprises individuellement et le virus qu'elles contiennent est amplifié sur cellules d'embryon de poulet. Leur ADN est ensuite extrait et analysé en comparaison avec l'ADN des 2 souches parentales de vaccine et de cowpox. Après digestion par l'enzyme EcoRI, les fragments d'ADN sont séparés par électrophorèse sur un gel d'agarose puis ils sont transférés à un filtre de nitrocellulose et hybridés au fragment SalI-K du virus de la vaccine marqué radioactivement au $^{32}$P.

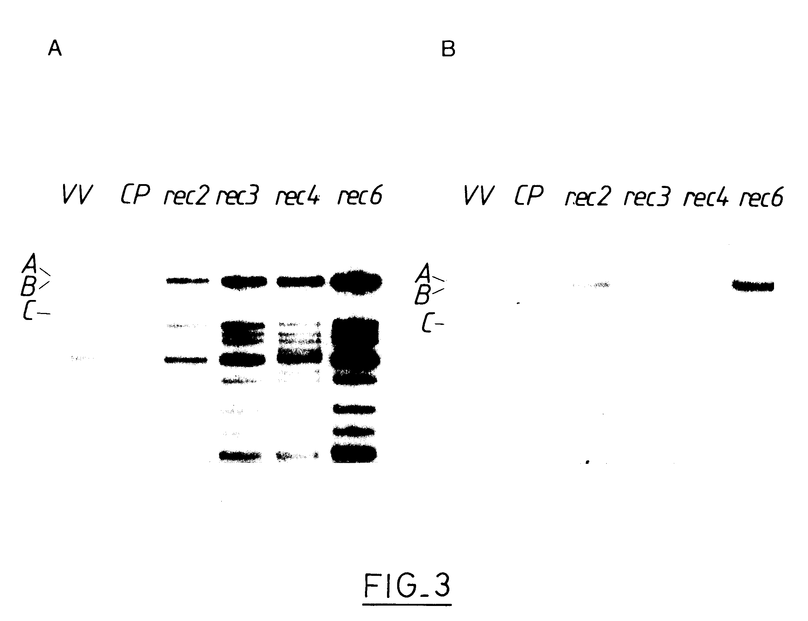

Après lavage de la nitrocellulose pour enlever la radioactivité fixée de manière aspécifique une autoradiographie est réalisée. L'autoradiographie (figure 3) montre que les recombinants du virus de la vaccine ayant intégré un fragment du cowpox ont perdu le fragment EcoRI-C typique du virus de la vaccine et comportent un fragment EcoRI qui s'hybride avec le fragment radioactif SalI-K de la vaccine ; ce fragment est intermédiaire en taille entre le fragment EcoRI-A du cowpox et le fragment EcoRI-C du virus de la vaccine.

Ce nouveau fragment EcoRI hybride cowpox-vaccine, présent dans tous les recombinants, provient d'une double recombinaison entre le fragment EcoRI-A du cowpox et le fragment EcoRI-C de la vaccine et doit renfermer l'information nécessaire pour la multiplication dans des cellules CHO. Pour que cette recombinaison ait pu se produire il fallait que l'information permettant la multiplication sur cellules CHO soit entourée de part et d'autre par des séquences du génome du cowpox homologues à des séquences du génome vaccine.

Exemple 3 Construction d'un plasmide recombinant portant la région du génome du cowpox qui permet la multiplication dans des cellules CHO.

Afin d'isoler l'information génétique permettant la multiplication dans des cellules CHO le fragment EcoRI-A de l'un des recombinants décrits dans l'exemple 2 a été cloné dans le plasmide bactérien pAT153 (Twigg et Sherratt, 1980).

L'ADN d'un des recombinants décrits dans l'exemple 2 est purifié puis coupé avec l'enzyme EcoRI. Le fragment EcoRI-A est élué d'un gel d'agarose puis inséré dans le plasmide pAT153 préalablement soumis à l'action d'EcoRI. Des bactéries HB101 sont transformées avec le mélange de ligation puis l'ADN des colonies obtenues est transféré sur nitrocellulose et hybridé au fragment SalI-K du virus de la vaccine. Les colonies positives à l'hybridation sont amplifiées et l'ADN plasmidique qu'elles contiennent est purifié. Deux plasmides ont été retenus : pEA1 et pEA2, qui correspondent à l'insertion du fragment EcoRI-A dans les deux orientations opposées dans le vecteur pAT153.

Pour vérifier que ces plasmides portent l'information génétique permettant la multiplication du virus sur cellules CHO on provoque une recombinaison entre l'insert d'ADN du plasmide et un virus de la vaccine : des cellules d'embryons de poulets sont infectées avec le mutant thermosensible du virus de la vaccine tsN7 à raison de 0,1 ufp par cellule puis transfectées avec l'ADN du virus de la vaccine sauvage et avec l'ADN du plasmide pEA1 ou pEA2. Des témoins sans ADN du virus de la vaccine ou sans plasmide sont également réalisés. Après 48 heures d'incubation à 39,5°C, les cellules sont congelées puis décongelées et le virus issu de l'infection est titré sur cellules CHO. Seuls les échantillons provenant de cellules transfectées avec l'ADN du virus de la vaccine et les plasmides pEA1 ou pEA2 donnent des plages sur cellules CHO.

Exemple 4 Sous-clonage de fragments de plus petite taille du pEA1 dans un plasmide vecteur destiné à la recombinaison avec le virus de la vaccine.

Pour affiner la localisation de l'information génétique permettant la multiplication dans des cellules CHO, des fragments de restriction recouvrant des portions réduites du plasmide pEA1 ont été clonés dans un plasmide portant le gène thymidine kinase (TK) du virus de la vaccine : pTG186poly.

- Construction de pTG186poly :

Le fragment HindIII (Hin-J) du génome du virus de la vaccine (VV) contient le gène complet de la thymidine kinase (TK) qui a déjà été utilisé précédemment pour permettre l'échange et la recombinaison d'un fragment d'ADN étranger dans le génome de VV (Mackett et al., 1982).

Il est important de noter que le transfert d'un insert dans le gène TK du génome de VV crée un virus TK déficient ce qui facilite sa sélection.

Il a tout d'abord été nécessaire de produire un plasmide de petite taille portant un site unique HindIII utilisable pour l'intégration du fragment Hin-J de VV. En outre, il était nécessaire d'éliminer les sites de restriction non nécessaires du plasmide, de façon à permettre les manipulations ultérieures.

La construction a été amorcée à partir du plasmide pML2 (Lusky et Botchan, 1981) qui est un vecteur dérivé du plasmide pBR322 dans lequel le segment compris entre les nucléotides 1089 et 2491 a été perdu par délétion spontanée. D'abord la séquence de PstI a été éliminée par insertion du fragment AhaIII-AhaIII de pUC8 (Vieira et Messing, 1982) entre deux sites AhaIII de pML2 en éliminant 19 paires de bases.
On a utilisé la méthode du "linker-tailing" (Lathe et al., 1984) pour insérer un adaptateur HindIII entre les sites NruI et EcoRI traité par S1 de ce plasmide, en éliminant le site BamHI. Ceci conduit à un plasmide de 2049 paires de bases portant le gène $\beta$-lactamase fonctionnel (conférant la résistance à l'ampicilline) et comportant en outre une origine de réplication active dans E. coli et un site de restriction unique HindIII. Cette construction a été appelée pTG1H.

Le fragment Hin-J de l'ADN de VV portant le gène TK a préalablement été cloné dans le vecteur pAT153 (Drillien et Spehner, 1983). Ce fragment de 4,6 kb a été recloné dans le site HindIII de pTG1H. Un clone a été sélectionné dans lequel le gène TK est situé distalement par rapport au gène codant pour la résistance à l'ampicilline. Cette construction a été appelée pTG1H-TK.

La construction pTG1H-TK a été utilisée comme vecteur pour les constructions suivantes.

L'étape suivante a été d'isoler un promoteur de VV utilisable pour commander l'expression du gène étranger à intégrer dans VV. Le promoteur d'un gène précoce codant pour une protéine de 7500 daltons (7,5 K) a déjà été utilisé avec succès dans un but identique (Smith et al., 1983) et on a donc procédé à l'isolement de ce segment.

Le gène 7,5 K est situé sur l'un des plus petits fragments SalI (fragment Sal-S) du génome de VV type WR (Venkatasan et al., 1981). Comme les petits fragments sont clonés de façon préférentielle, une grande proportion des clones obtenus par clonage direct de l'ADN de VV type WR coupé par SalI dans le plasmide pBR322 porte le fragment Sal-S. Ce fragment est transféré sur le bactériophage vecteur M13mp701 (Kieny et al., 1983), par digestion SalI et religation, ce qui donne le phage M13.TG.Sal-S.

Dans ce clone, un site ScaI se trouve immédiatement à proximité de l'ATG d'initiation du gène 7,5 K. En aval du gène 7,5 K se trouvent situés des sites uniques BamHI et EcoRI provenant du vecteur. Les sites BamHI et ScaI sont fusionnés par l'intermédiaire d'un adaptateur BglII : 5'-CAGATCTG-3' après avoir complété les extrémités générées par digestion BamHI avec le fragment Klenow de la polymérase de E. coli. Ce procédé élimine le site ScaI mais reconstitue le site BamHI et déplace le site unique EcoRI en eval. En même temps, le site SalI (AccI) en aval est éliminé et le site SalI en amont devient donc unique. Cette construction est appelée M13.TG.7,5K.

A l'intérieur du fr agment Hin-J de l'ADN de VV se trouvent situés des sites ClaI et EcoRI qui sont séparés par environ 30 paires de bases (Weir et Moss, 1983). Le fragment promoteur de 7,5 K présent dans M13.TG.7,5K est excisé par AccI et EcoRI et cloné entre les sites ClaI et EcoRI de pTG1-H-TK pour générer pTG1H-TK-P7,5K.

Dans cette construction les sites BamHI et EcoRI uniques du vecteur M13 se retrouvent immédiatement en aval de la séquence du promoteur 7,5 K. Ces sites uniques BamHI et EcoRI seront utilisés dans la construction suivante.

Le segment polylinker du bactériophage M13TG131 (Kieny et al., 1983) est excisé par EcoRI et BglII et inséré entre les sites EcoRI et BamHI du plasmide pTG1H-TK-P7,5K, générant le pTG186poly. Dans cette construction, 5 sites de restriction uniques sont disponibles pour le clonage d'un gène étranger sous le contrôle du promoteur P7,5K : PstI, BamHI, SstI, SmaI et EcoRI.

- Insertion de fragments du pEA1 dans pTG186poly :

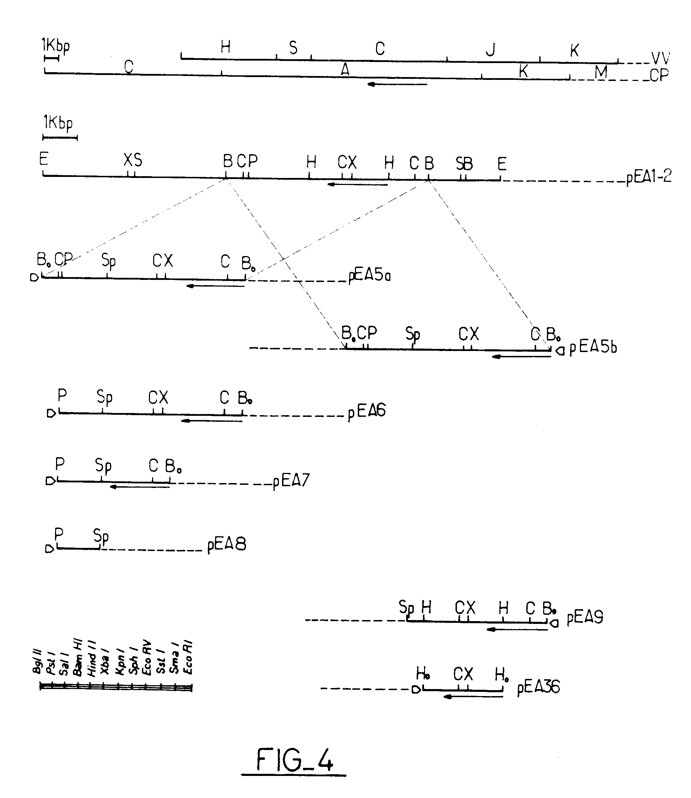

Le fragment EcoRIA du plasmide pEA1 a été digéré par différents enzymes et des fragments de différentes tailles ont été insérés dans le pTG186poly.
Les plasmides recombinants dérivés de pTG186poly et contenant des portions du fragment EcoRI-A du plasmide recombinant pEA1 sont représentés dans la figure 4.

La coupure du plasmide pEA1 avec l'enzyme BglII donne plusieurs fragments. Le plus grand des fragments BglII a été inséré dans le site BamHI du vecteur pTG186poly pour donner les plasmides pEA5a et pEA5b qui se distinguent par l'orientation de l'insertion.

Le plasmide pEA6 est dérivé du plasmide pEA5a par coupure de ce dernier avec l'enzyme PstI puis recircularisation du plus grand fragment ce qui a pour effet de déléter le petit fragment BglII-PstI du plasmide pEA5a.

Le plasmide pEA7 provient de pEA6 après coupure de ce dernier avec l'enzyme ClaI et recircularisation du grand fragment obtenu ce qui donne une délétion du petit fragment ClaI.

Le plasmide pEA8 provient de pEA6 après coupure de ce dernier par SphI puis recircularisation du grand fragment ce qui donne une délétion du fragment SphI-BglII de droite.

Le plasmide pEA9 provient du plasmide pEA5b après coupure de ce dernier par SphI puis religation ce qui donne une délétion du fragment gauche SphI-BglIII.

Le plasmide pEA36 a été construit en deux étapes. D'abord le petit fragment HpaI a été isolé du plasmide pEA9 et il a été inséré dans le site SmaI du vecteur M13-130. Puis le fragment HpaI a été sorti du vecteur M13-130 grâce aux sites EcoRI et PstI et il a été intégré dans les sites EcoRI et PstI du vecteur pTG186poly.

Chacun de ces plasmides a été utilisé dans une expérience de transfection de manière à transférer l'insertion qui se trouve dans le gène TK porté par le plasmide au génome du virus de la vaccine, selon la méthode décrite dans l'exemple 3.

Les plasmides capables de conférer la capacité de multiplication dans des cellules CHO au virus de la vaccine sont pEA1, 2, 5a, 5b, 6, 9, 36.

Le plus petit fragment ayant cette propriété est porté par le plasmide pEA36. Il comprend 2004 paires de bases.

Exemple 5 Séquençage du gène permettant la multiplication du virus de la vaccine dans les cellules CHO.

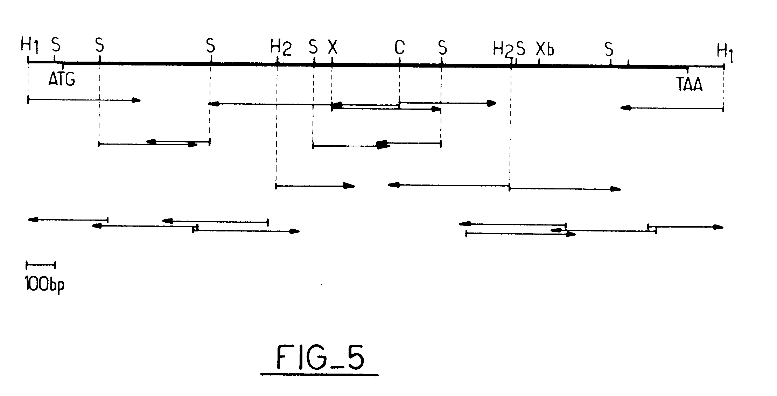

L'ADN du cowpox porté par le plasmide pEA36 et situé entre les sites HpaI a été entièrement séquencé selon la méthode des didéoxynucléotides (Sanger et al. 1980) après insertion des plus petits fragments dans les phages M13-130 et M13-131 (Kieny et al. 1983) en suivant la stratégie schématisée dans la figure 5.

Le cadre de lecture pouvant coder pour une protéine de 77.000 daltons débute aux nucléotides ATG et se termine aux nucléotides TAA représentés sur la carte de la figure 5. Les flèches en-dessous de la carte indiquent l'endroit où commence la lecture de chaque clone, la longueur de la lecture et sa direction. Lorsque le début de lecture ne commence pas à un site de restriction, des amorces constituées d'oligonucléotides synthétiques déduits d'une séquence partielle ont été utilisées.

La séquence complète du gène ainsi que la séquence de la protéine de 77.000 daltons pour laquelle il peut coder est présentée dans la figure 6.

Exemple 6 Recombinaison homologue et intégration du gène du cowpox dans l'ADN de la vaccine, en dehors du gène TK.

Parmi les virus recombinants, décrits dans l'exemple 4, capables de se multiplier sur cellules CHO, on constate que certains sont TK$^+$ et d'autres TK$^-$.

Par exemple dans le transfection des cellules avec le plasmide pEA9, sur 27 plages de virus recombinants sélectionnées pour leur multiplication sur CHO, 16 étaient TK$^-$ et 11 TK$^+$.

La recombinaison introduisant l'information qui permet la multiplication dans des cellules CHO s'est donc produite dans une région différente de celle du gène qui code pour la thymidine kinase.

Ce résultat implique la possibilité de sélection pour l'insertion d'un gène étranger dans une région du génome autre que le gène TK, simultanément à la sélection pour la multiplication sur cellules CHO. Il suffit pour cela de juxtaposer le gène choisi et le gène permettant la croissance sur cellules CHO. L'insertion de ce dernier par recombinaison homologue avec le génome du virus de la vaccine entrainera la co-intégration du gène étranger.

Dépôt de souche représentative de l'invention.

La souche de E. coli 1106 portant le plasmide pEA36 a été déposée à la Collection Nationale de Cultures de Microorganismes sous le n° I 594, le 2 septembre 1986.

Le plasmide pEA36 porte un fragment d'ADN de 2.004 pb du virus du Cowpox qui permet la multiplication du virus dans les cellules CHO ; ce fragment d'ADN est destiné à la recombinaison in vivo avec le virus de la vaccine (qui ne se multiplie pas dans les cellules CHO). Par transfection des cellules avec l'ADN plasmidique et co-infection avec le virus de la vaccine, on pourra sélectionner des virus recombinants de la vaccine qui ont acquis la capacité de se multiplier sur cellules CHO.

REFERENCES

Drillien, R., Spehner, D. & Kirn, A. J. Virol. 28, 843-850 (1978).

Drillien, R., Spehner, D. & Kirn, A. Virology 119, 372-381 (1982).

Drillien, R. & Spehner, D. Virology 131, 385-393 (1983).

Kieny, M.P., Lathe, R. & Lecocq, J-P. Gene 26, 91-99 (1983).

Kieny, M.P., Lathe, R., Drillien, R., Spehner, D., Skory, S., Schmitt, D., Wiktor, T., Koprowski, H. & Lecocq. J-P. Nature 312, 163-166 (1984).

Lathe, R., Kieny, M.P., Schmitt, D., Curtis, P. and Lecocq, J-P. J. Molec. Appl. Genet. 2, 331-342 (1984).

Lusky & Botchon Nature 293, 79-81 (1981).

Mackett, M. & Archard, L.C. (1979) J. Gen. Virol. 45, 683-701 (1979).

Mackett, M., Smith, G.L. & Moss, B. P.N.A.S. (USA) 79, 7415-7419 (1982).

Maniatis, T., Fritsch, E.F. & Sambrook, J. "Molecular Cloning : A Laboratory Manual" Cold Spring Harbor Laboratory, Cold Spring Harbor, N.Y. (1982).

Moss, B. In "Virology" (B.N. Fields, ed.) pp. 685-703. Raven Press, New York.

Panicali, D. & Paoletti, E. P.N.A.S. (USA) 79, 4927-4931 (1982).

Panicali, D., Davis, S.W., Weinberg, R.L. & Paoletti, E. P.N.A.S. (USA) 80, 5364-5368 (1983).

Sanger, F.S., Nicklen, S. and Conlson, A.R. P.N.A.S. (USA) 74, 5463-5467 (1977).

Smith G.L., Mackett, M. & Moss, B. Nature 302, 490-495 (1983).

Smith, G.L., Murphy, B.R. & Moss, B. P.N.A.S. (USA) 80, 7155-7159 (1983).

Twigg, A.J. & Sherratt, D. Nature 283, 216-218 (1980).

Venkatasan, S., Baroudy, B.M. & Moss, B. Cell 25, 805-813 (1981).

Viera and Messing Gene 19, 259-268 (1982).

Weir, J.P. & Moss, B. J. Virol. 46, 530-537 (1983).

Légende des figures

Figure 1 Cartes de restriction de l'ADN de recombinants entre le virus de la vaccine et le cowpox.

Les quatre lignes horizontales supérieures représentent les cartes de restriction du virus de la vaccine (VV) et du cowpox (CP) obtenues après coupure avec les enzymes HindIII et XhoI. Les dix lignes horizontales suivantes représentent les génomes des recombinants isolés selon l'exemple 1 entre le VV et le CP. Les sites de restriction notés avec une flèche sont des sites caractéristiques du génome du cowpox ; ceux notés avec un trait vertical sont caractéristiques du génome du virus de la vaccine. Les sites communs aux deux génomes ne sont pas notés. Les pointillés indiquent des régions d'incertitude dans la détermination.

Figure 2 Cartes de restriction de l'extrémité gauche des virus de la vaccine (VV) et du cowpox (CP) après coupure avec les enzymes EcoRI, HindIII et Sal1.

Les fragments sont notés conventionnellement A, B, C ..., par ordre de taille décroissant.

Figure 3 Caractérisation des profils de restriction de l'ADN des recombinants entre le virus de la vaccine et le cowpox.

L'ADN du virus de la vaccine (VV), du cowpox (CP) et de quatre recombinants appelés rec2, rec3, rec4 et rec6, isolés selon l'exemple 2, a été préparé à partir de cellules d'embryon de poulet infectées en présence de thymidine $^3$H.

La partie A de la figure présente une autoradiographie des fragments obtenus après digestion de l'ADN avec EcoRI, séparation des fragments sur gel d'agarose et autoradiographie du gel sec pendant quinze jours d'exposition. En B, l'ADN traité de la même manière qu'en A a été transféré sur nitrocellulose et hybridé à une sonde Sal1-K portée par le plasmide pAT153.

Figure 4 Plasmides recombinants contenant des portions du fragment EcoRIA du plasmide pEA1 intégrés dans le vecteur pTG186poly.

Les deux lignes horizontales supérieures représentent l'extrémité gauche de l'ADN du virus de la vaccine et du cowpox avec les sites de restriction pour l'enzyme EcoRI. La troisième ligne est une représentation linéaire du plasmide pEA1 avec le fragment EcoRI-A en trait continu et le plasmide pAT153 en trait discontinu.

Les lignes suivantes sont des représentations linéaires des différents plasmides recombinants avec le fragment dérivé de pEA1 en trait continu et le vecteur pTG186poly en traits discontinus ; le promoteur du gène 7.5 K est symbolisé par une flèche ouverte qui donne le sens de la transciption.

Les sites de restriction sont symbolisés par les lettres suivantes :

| | |
|---|---|
| B | BglII |
| C | ClaI |
| E | EcoRI |
| H | HapI |
| S | Sal1 |
| Sp | Sph1 |
| X | Xho1 |

Figure 5 Stratégie de séquençage du gène permettant la multiplication dans des cellules CHO.

La ligne horizontale en trait gras représente le fragment d'ADN séquencé et compris entre les sites HpaI (H1). Les autres sites de restriction sont symbolisés par les lettres suivantes S (Sau3A), H2 (Hpa2), X (XhoI), C (ClaI), Xb (XbaI). Le cadre de lecture pouvant coder pour une protéine de 80.000 daltons débute aux nucléotides ATG et se termine aux nucléotides TAA portés sur la carte. Les flèches en-dessous de la carte indiquent l'endroit où commence la lecture de chaque clone, la longueur de la lecture et sa direction.

Figure 6 Séquence du gène permettant la multiplication dans les cellules CHO.

**Revendications**

**Revendications pour les Etats contractants suivants : BE, CH, DE, FR, GB, IT, LI, LU, NL, SE**

1. Fragment d'ADN isolé du virus du cowpox participant à la multiplication de ce virus dans les cellules d'ovaires de hamster chinois (CHO), qui code pour une protéine comprenant une séquence en acides aminés substantiellement telle que montrée à la figure 6 ou pour une portion fonctionnelle de cette protéine.

2. Fragment d'ADN selon la revendication 1, qui code pour une protéine comprenant une séquence en acides aminés telle que montrée à la figure 6.

3. Un virus de la vaccine capable de se multiplier dans des cellules CHO, dans le génome duquel est inséré un premier fragment d'ADN selon la revendication 1 ou 2, ledit fragment d'ADN étant placé sous le contrôle de régions appropriées assurant son expression.

4. Un virus de la vaccine selon la revendication 3, dans le génome duquel est inséré un premier fragment d'ADN codant pour protéine comprenant une séquence en acides aminés telle que montrée à la figure 6 ; ledit fragment d'ADN étant placé sous le contrôle de régions natives du virus du cowpox assurant son expression.

5. Un virus de la vaccine selon la revendication 3 ou 4, dans lequel le premier fragment d'ADN est inséré dans une région du génome de la vaccine qui présente un haut degré d'homologie avec ledit premier fragment d'ADN.

6. Un virus de la vaccine selon l'une des revendications 3 à 5, dans le génome duquel est inséré un second fragment d'ADN codant pour une protéine d'intérêt industriel ; ledit second fragment d'ADN étant placé sous le contrôle de régions appropriées assurant son expression.

7. Culture de cellules CHO infectées par un virus de la vaccine selon l'une des revendications 3 à 6.

8. Culture de cellules CHO infectées par un virus de la vaccine selon la revendication 6.

9. Cellule CHO dans le génome de laquelle est inséré un fragment d'ADN selon la revendication 1 ou 2 ; ledit fragment étant placé sous le contrôle de régions appropriées assurant son expression.

10. Cellule CHO selon la revendication 9, dans le génome de laquelle est inséré un fragment d'ADN qui code pour une protéine comprenant une séquence en acides aminés telle que montrée à la figure 6 ; ledit fragment d'ADN étant placé sous le contrôle de régions natives du virus du cowpox assurant son expression.

11. Culture de cellules CHO selon la revendication 9 ou 10, qui est infectée par un virus de la vaccine.

12. Culture de cellules CHO selon la revendication 9 ou 10, qui est infectée par un virus de la vaccine dans le génome duquel est inséré un fragment d'ADN codant pour une protéine d'intérêt industriel placé sous le contrôle de régions appropriées assurant son expression.

13. Procédé de production d'une protéine d'intérêt industriel qui comprend l'acte de récolter ladite protéine à partir d'une culture de cellules selon la revendication 8 ou 12.

**Revendications pour les Etats contractants suivants : AT, ES, GR**

1. Procédé de production d'une protéine d'intérêt industriel qui comprend l'acte de récolter ladite protéine à partir d'une culture de cellules d'ovaires de hamster chinois (CHO) infectées par un virus de la vaccine capable de se multiplier dans des cellules de CHO, dans le génome duquel est inséré un premier fragment d'ADN isolé du virus du cowpox participant à la multiplication de ce virus dans les cellules de CHO, qui code pour une protéine comprenant une séquence en acide aminé substantiellement telle que montrée à la figure 6 ou pour une portion fonctionnelle de cette protéine, ledit premier fragment d'ADN étant placé sous le contrôle de régions appropriées assurant son expression et un second fragment d'ADN codant pour une protéine d'intérêt industriel, ledit second fragment d'ADN étant placé sous le contrôle de régions appropriées assurant son expression.

2. Procédé selon la revendication 1, caractérisé en ce que le premier fragment d'ADN code pour une protéine comprenant une séquence en acides aminés telle que montrée à la figure 6.

3. Procédé selon l'une des revendications 1 ou 2, caractérisé en ce que le premier fragment d'ADN est placé sous le contrôle des régions natives du virus du cowpox assurant son expression.

4. Procédé selon l'une des revendications 1 à 3, caractérisé en ce que le premier fragment d'ADN est inséré dans une région du génome de la vaccine qui présente un haut degré d'hommologie avec ledit premier fragment d'ADN.

**Claims**

**Claims for the following Contracting States : BE, CH, DE, FR, GB, IT, LI, LU, NL, SE**

1. DNA fragment isolated from cowpox virus which participates in the multiplication of this virus in chinese hamster ovarian (CHO) cells, coding for a protein comprising an aminoacids sequence substantially such as shown in figure 6 or a function equivalent portion of this protein.

2. The DNA fragment as claimed in claim 1, coding for a protein comprising an aminoacids sequence such as shown in figure 6.

3. The vaccinia virus capable of multiplicate in CHO cells, in which genome is inserted a first DNA fragment according to claim 1 or 2, said DNA fragment being under the control of appropriate regions which provide for its expression.

4. A vaccinia virus as claimed in claim 3, in which genome is inserted a first DNA fragment coding for a protein comprising an aminoacids sequence such as shown in figure 6 ; said DNA fragment being under the control of native regions of the cowpox virus which provide for its expression.

5. The vaccinia virus as claimed in claim 3 or 4, in which the first DNA fragment is inserted in a region of the genome of the vaccinia virus showing a high degree of homology with said first DNA fragment.

6. The vaccinia virus as claimed in one of the claims 3 to 5, in which genome is inserted a second DNA fragment coding for a protein of industrial value; said second fragment being under the control of appropriate regions which provide for its expression.

7. A culture of CHO cells infected with a vaccinia virus as claimed in one of the claims 3 to 6.

8. A culture of CHO cells infected with a vaccinia virus as claimed in claim 6.

9. A CHO cell in which genome is inserted a DNA fragment as claimed in claims 1 or 2; said fragment being under the control of appropriate regions which provide for its expression.

10. The CHO cell as claimed in claim 9, in which genome is inserted a DNA fragment coding for a protein comprising an aminoacids sequence such as shown in figure 6; said DNA fragment being under the control of native regions of the cowpox virus which provide for its expression.

11. A culture of CHO cells as claimed in claim 9 or 10, which is infected with a vaccinia virus.

12. A culture of CHO cells as claimed in claim 9 or 10, which is infected with a vaccinia virus in which genome is inserted a DNA fragment coding for a protein of industrial value being under the control of appropriate regions which provide for its expression.

13. A process for the production of a protein of industrial value which comprises the action of collecting said protein from a culture of cells as claimed in claim 8 or 12.

**Claims for the following Contracting States : AT, ES, GR**

1. Process for the production of a protein of industrial value which comprises the action of collecting said protein from a culture of chinese hamster ovarian (CHO) cells infected with a vaccinia virus capable of multiplicate in CHO cells, in which genome is inserted a first DNA fragment isolated from cowpox virus which participates in the multiplication of this virus in CHO cells, coding for a protein comprising an aminoacids sequence substantially such as shown in figure 6 or a function equivalent portion of this protein, said first DNA fragment being under the control of appropriate regions which provide for its expression and a second DNA fragment coding for a protein of industrial value, said second fragment being under the control of appropriate regions which provide for its expression.

2. The process as claimed in claim 1, characterized in that the first DNA fragment is coding for a protein comprising an aminoacid sequence such as shown in figure 6.

3. The process as claimed in claim 1 or 2, characterized in that the first DNA fragment is under the control of native regions of the cowpox virus which provide for its expression.

4. The process as claimed in one of the claims 1 to 3, characterized in that the first DNA fragment is inserted in a region of the genome of the vaccinia virus which presents a high degree of homology with said first DNA fragment.

**Patentansprüche**

**Patentansprüche für folgende Vertragsstaaten : BE, CH, DE, FR, GB, IT, LI, LU, NL, SE**

1. DNA-Fragment, isoliert aus dem Kuhpockenvirus, das an der Vermehrung dieses Virus in den Ovarienzellen des chinesischen Hamsters (CHO) teilnimmt, das für ein Protein, umfassend eine Aminosäuresequenz, im wesentlichen wie in der Figur 6 dargestellt, oder für einen funktionellen Teil dieses Proteins, kodiert.

2. DNA-Fragment nach Anspruch 1, das für ein Protein kodiert, das eine Aminosäuresequenz, wie in der Figur 6 dargestellt, umfaßt.

3. Ein Kuhpockenvirus, das sich in CHO-Zellen vermehren kann, in dessen Genom ein erstes DNA-Fragment nach Anspruch 1 oder 2 eingeführt ist, wobei dieses DNA-Fragment unter die Kontrolle von geeigneten Regionen plaziert ist, die dessen Expression sicherstellen.

4. Ein Kuhpockenvirus nach Anspruch 3, in dessen Genom ein erstes DNA-Fragment eingeführt ist, das für Protein kodiert, das eine Aminosäuresequenz, wie in der Figur 6 dargestellt, umfaßt; wobei dieses DNA-Fragment unter die Kontrolle von nativen Regionen des Kuhpokkenvirus plaziert ist, die seine Expression sicherstellen.

5. Ein Kuhpockenvirus nach Anspruch 3 oder 4, in dem das erste DNS-Fragment in eine Genom-Region der Kuhpocken eingeführt ist, die ein hohes Homologie-Ausmaß mit diesem ersten DNA-Fragment aufweist.

6. Ein Kuhpockenvirus nach einem der Ansprüche 3 bis 5, in dessen Genom ein Zweites DNA-Fragment eingeführt ist, das für ein Protein von industriellem Interesse kodiert; wobei dieses zweite DNA-Fragment unter die Kontrolle von geeigneten Regionen plaziert ist, die seine Expression sicherstellen.

7. CHO-Zellkultur, infiziert mit einem Kuhpockenvirus nach einem der Ansprüche 3 bis 6.

8. CHO-Zellkultur, infiziert mit einem Kuhpockenvirus nach Anspruch 6.

9. CHO-Zelle in deren Genom ein DNA-Fragment nach Anspruch 1 oder 2 eingeführt ist; wobei dieses Fragment unter die Kontrolle von geeigneten Regionen, die seine Expression sicherstellen, plaziert ist.

10. CHO-Zelle nach Anspruch 9, in deren Genom ein DNA-Fragment eingeführt ist, das für ein Protein kodiert, das eine Aminosäuresequenz, wie in Figur 6 dargestellt, kodiert; wobei dieses DNA-Fragment unter die Kontrolle von nativen Regionen des Kuhpockenvirus plaziert ist, die seine Expression sicherstellen.

11. CHO-Zellkultur nach Anspruch 9 oder 10, die mit einem Kuhpockenvirus infiziert ist.

12. CHO-Zellkultur nach Anspruch 9 oder 10, die mit einem Kuhpockenvirus infiziert ist, in dessen Genom ein DNA-Fragment eingeführt ist, das für ein Protein von industriellem Interesse kodiert, das unter die Kontrolle von geeigneten Regionen plaziert ist, die seine Expression sicherstellen.

13. Verfahren zur Herstellung eines Proteins von industriellem Interesse, welches die Ernte des Proteins aus einer Zellkultur nach den Ansprüchen 8 oder 12 umfaßt.

**Patentansprüche für folgende Vertragsstaaten : AT, ES, GR**

1. Verfahren zur Herstellung eines Proteins von industriellem Interesse, umfassend die Ernte dieses Proteins aus einer Kultur von Ovarienzellen des chinesischen Hamsters (CHO), die mit einem Kuhpockenvirus infiziert sind, das geeignet ist sich in den CHO-Zellen zu vermehren, in dessen Genom ein erstes DNA-Fragment, isoliert aus dem Kuhpockenvirus, eingeführt ist, das an der Vermehrung dieses Virus in den CHO-Zellen teilnimmt, das für ein Protein, welches eine Aminosäuresequenz, im wesentlichen wie in der Figur 6 dargestellt, oder für einen funktionellen Teil dieses Proteins, kodiert, wobei dieses erste DNA-Fragment unter die Kontrolle von geeigneten Regionen plaziert ist, die seine Expression sicherstellen, sowie ein zweites DNA-Fragment, das für ein Protein von industriellem Interesse kodiert, wobei das zweite DNA-Fragment unter die Kontrolle von geeigneten Regionen plaziert ist, die seine Expression sicherstellen.

2. Verfahren nach Anspruch 1, dadurch gekennzeichnet, daß das erste DNA-Fragment für ein Protein kodiert, das eine Aminosäuresequenz, wie in Figur 6 dargestellt, umfaßt.

3. Verfahren nach einem der Ansprüche 1 oder 2, dadurch gekennzeichnet, daß das erste DNA-Fragment unter die Kontrolle von nativen Regionen des Kuhpockenvirus plaziert ist, die seine Expression sicherstellen.

4. Verfahren nach einem der Ansprüche 1 bis 3, dadurch gekennzeichnet, daß das erste DNA-Fragment in eine Region des Genoms der Kuhpocken eingeführt ist, die ein hohes Homologie-Ausmaß mit diesem ersten DNA-Fragment aufweist.

FIG_1

FIG_2

EP 0 262 043 B1

A                                    B

VV  CP rec2 rec3 rec4 rec6          VV  CP  rec2 rec3 rec4 rec6

A>                                  A>
B>                                  B>
C–                                  C–

FIG_3

FIG_4

15

FIG_5

EP 0 262 043 B1

30 60 90
GTG TTA ACG GAG GTG TAA ACT CTG GAG TTA ATA AAA TTT AAA CAC TAA ATT ATT TTT TAT TAA TAA TTG TAC AAG TTT TTG ATC TGG TAT

120 150 180
AAA TAC ATT CAA AAA TGA TAA TTT AAT GAC ATT AGT TGT GCG GGT GTA TAG AGT TCA CAG TAG CTC ATT CAC TTC TAT TCA GTC AAA ATG
                                                                                                                        Met

210 240 270
TTT GAT TAT CTG GAA AAT GAG GAG GTG GCT CTC GAT GAA CTT AAA CAG ATG TTG AGA GAT AGA GAT CCT AAT GAT ACC AGG AAC CAA TTC
Phe Asp Tyr Leu Glu Asn Glu Glu Val Ala Leu Asp Glu Leu Lys Gln Met Leu Arg Asp Arg Asp Pro Asn Asp Thr Arg Asn Gln Phe

300 330 360
AAG AAT AAT GCT CTA CAT GCA TAC CTT TTC AAT GAG CAC TGT AAT AAT GTT GAG GTT GTC AAA CTA CTA CTA GAC AGT GGT ACT AAT CCA
Lys Asn Asn Ala Leu His Ala Tyr Leu Phe Asn Glu His Cys Asn Asn Val Glu Val Val Lys Leu Leu Leu Asp Ser Gly Thr Asn Pro

390 420 450
TTA CAC AAA AAT TGG AGA CAG CTT ACT CCA TTA GGG GAA TAC ACA AAT AGT AGA CAT GGT AAA GTT AAT AAG GAT ATA GCG ATG GTT CTA
Leu His Lys Asn Trp Arg Gln Leu Thr Pro Leu Gly Glu Tyr Thr Asn Ser Arg His Gly Lys Val Asn Lys Asp Ile Ala Met Val Leu

480 510 540
CTA GAA GCT ACT GGA TAT AGC AAC ATA AAT GAC TTT AAT ATA TTC ACC TAT ATG AAA TCC AAA AAT GTA GAT ATT GAC TTG ATA AAG GTA
Leu Glu Ala Thr Gly Tyr Ser Asn Ile Asn Asp Phe Asn Ile Phe Thr Tyr Met Lys Ser Lys Asn Val Asp Ile Asp Leu Ile Lys Val

FIG_6

EP 0 262 043 B1

```
                              570                                                  600                                                  630
TTG GTA GAA CAT GGA TTT GAT TTT AGT GTT AAA TGC GAA AAA CAT CAT TCA GTT ATA GAA AAT TAT GTA ATG ACA GAT GAT CCT GTT CCT
Leu Val Glu His Gly Phe Asp Phe Ser Val Lys Cys Glu Lys His His Ser Val Ile Glu Asn Tyr Val Met Thr Asp Asp Pro Val Pro

                              660                                                  690                                                  720
GAA ATT ATT GAT TTA TTC ATA GAA AAT GGA TGC AGT GTT ATT TAT GAG GAC GAG GAT GAT GAG TAC GGA TAC GCG TAT GAA GAA TAT CAC
Glu Ile Ile Asp Leu Phe Ile Glu Asn Gly Cys Ser Val Ile Tyr Glu Asp Glu Asp Asp Glu Tyr Gly Tyr Ala Tyr Glu Glu Tyr His

                              750                                                  780                                                  810
TCA CAA AAT GAC GAT TAT CAA CCA CGA AAT TGC GGT ACA GTA TTA CAT CTG TAT ATC ATC TCT CAT CTG TAT TCA GAG TCG GAT TCG AGA
Ser Gln Asn Asp Asp Tyr Gln Pro Arg Asn Cys Gly Thr Val Leu His Leu Tyr Ile Ile Ser His Leu Tyr Ser Glu Ser Asp Ser Arg

                              840                                                  870                                                  900
TCA TGT GTG AAC CCG GAA GTT GTT AAA TGT CTG ATT AAT CAT GGA ATC AAC CCA TCT TCT ATA-GAT AAA AAC TAT TGT ACA GCT CTT CAA
Ser Cys Val Asn Pro Glu Val Val Lys Cys Leu Ile Asn His Gly Ile Asn Pro Ser Ser Ile Asp Lys Asn Tyr Cys Thr Ala Leu Gln

                              930                                                  960                                                  990
TAT TAT ATT AAG TCA TCT CAT ATA GAT ATA GAC ATC GTT AAA TTG TTA ATG AAA GGA ATA GAT AAC ACG GCT TAT TCA TAT ATA GAC GAT
Tyr Tyr Ile Lys Ser Ser His Ile Asp Ile Asp Ile Val Lys Leu Leu Met Lys Gly Ile Asp Asn Thr Ala Tyr Ser Tyr Ile Asp Asp

                             1020                                                 1050                                                 1080
CTA ACA TGT TGT ACT CGA GGA ATT ATG GCT GAT TAT CTA AAT AGT GAT TAT AGA TAC AAT AAA GAT GTA GAT TTA GAT TTG GTC AAA TTG
Leu Thr Cys Cys Thr Arg Gly Ile Met Ala Asp Tyr Leu Asn Ser Asp Tyr Arg Tyr Asn Lys Asp Val Asp Leu Asp Leu Val Lys Leu
```

# FIG. 6

```
                        1110                              1140                              1170
TTT TTG GAG AAT GGA AAA CCG CAC GGA ATA ATG TGT AGT ATT GTA CCA CTA TGG AGA AAT GAT AAG GAA ACC ATC TCT TTG ATA TTG AAA
Phe Leu Glu Asn Gly Lys Pro His Gly Ile Met Cys Ser Ile Val Pro Leu Trp Arg Asn Asp Lys Glu Thr Ile Ser Leu Ile Leu Lys

                        1200                              1230                              1260
ACA ATG AAC TCG GAT GTC CTC CAA CAT ATA CTA ATT GAG TAT ATA ACA TTC AGC GAT ATC GAT ATC TCT CTA GTG GAA TAC ATG TTG GAA
Thr Met Asn Ser Asp Val Leu Gln His Ile Leu Ile Glu Tyr Ile Thr Phe Ser Asp Ile Asp Ile Ser Leu Val Glu Tyr Met Leu Glu

                        1290                              1320                              1350
TAT GGA GCT GTG GTA AAT AAA GAG GCT ATT CAC GGA TAC TTT AAA AAT ATT AAT ATT GAT TCT TAC ACG ATG AAA TAT CTA CTA AAA AAG
Tyr Gly Ala Val Val Asn Lys Glu Ala Ile His Gly Tyr Phe Lys Asn Ile Asn Ile Asp Ser Tyr Thr Met Lys Tyr Leu Leu Lys Lys

                        1380                              1410                              1440
GAA GGG GGA GAT GCC GTC AAT CAT CTC GAT GAT GGA GAG ATC CCG ATT GGA CAC CTA TGT AAA TCC AAC TAT GGA CGT TAT AAT TTC TAC
Glu Gly Gly Asp Ala Val Asn His Leu Asp Asp Gly Glu Ile Pro Ile Gly His Leu Cys Lys Ser Asn Tyr Gly Arg Tyr Asn Phe Tyr

                        1470                              1500                              1530
ACT GAT ACA TAC AGA CAG GGT TTT CGT GAT ATG TCT TAT GCT TGC CCA ATT CTT AGT ACT ATA AAC ATT TGC CTA CCT TAT CTT AAA GAC
Thr Asp Thr Tyr Arg Gln Gly Phe Arg Asp Met Ser Tyr Ala Cys Pro Ile Leu Ser Thr Ile Asn Ile Cys Leu Pro Tyr Leu Lys Asp

                        1560                              1590                              1620
ATT AAC ATG ATT GAC AAA CGA GGA GAA ACA CTT CTT CAC AAG GCT GTT AGA TAT AAT AAA CAA TCT CTA GTG TCT TTA CTG CTA GAA TCC
Ile Asn Met Ile Asp Lys Arg Gly Glu Thr Leu Leu His Lys Ala Val Arg Tyr Asn Lys Gln Ser Leu Val Ser Leu Leu Leu Glu Ser

                        1650                              1680                              1710
GGT TCA GAT GTC AAC ATT AGA TCA AAT AAC GGA TAT ACA TGT ATA GCC ATT GCA ATC AAC GAA TCT AGA AAC ATT GAA CTG CTG AAC ATG
Gly Ser Asp Val Asn Ile Arg Ser Asn Asn Gly Tyr Thr Cys Ile Ala Ile Ala Ile Asn Glu Ser Arg Asn Ile Glu Leu Leu Asn Met
```

# FIG_6

EP 0 262 043 B1

EP 0 262 043 B1

```
                    1740                                      1770                                     1800
CTA TTA TGT CAT AAA CCT ACA TTA GAT TGT GTG ATT GAT TCA TTG AGA GAA ATA TCT AAC ATA GTA GAT AAT GCC TAT GCT ATA AAA CAA
Leu Leu Cys His Lys Pro Thr Leu Asp Cys Val Ile Asp Ser Leu Arg Glu Ile Ser Asn Ile Val Asp Asn Ala Tyr Ala Ile Lys Gln

                    1830                                      1860                                     1890
TGT ATT AGA TAT GCC ATG ATT ATA GAT GAC TGT ATA TCG TCT AAG ATT CCA GAG TCC ATA AGT AAA CAC TAT AAT GAT TAT ATA GAT ATT
Cys Ile Arg Tyr Ala Met Ile Ile Asp Asp Cys Ile Ser Ser Lys Ile Pro Glu Ser Ile Ser Lys His Tyr Asn Asp Tyr Ile Asp Ile

                    1920                                      1950                                     1980
TGC AAT CAA GAA TTG AAC GAG ATG AAA AAA ATA ATA GTG GGA GGC AAC ACT ATG TTC TCA TTA ATA TTT ACT GAT CAT GGA GCT AAA ATT
Cys Asn Gln Glu Leu Asn Glu Met Lys Lys Ile Ile Val Gly Gly Asn Thr Met Phe Ser Leu Ile Phe Thr Asp His Gly Ala Lys Ile

                    2010                                      2040                                     2070
ATT CAT CGG TAT GCC AAT AAT CCA GAA TTA CGT GCG TAT TAT GAG TCA AAA CAA AAT AAA ATA TAC GTG GAA GTA TAT GAT ATT ATT TCC
Ile His Arg Tyr Ala Asn Asn Pro Glu Leu Arg Ala Tyr Tyr Glu Ser Lys Gln Asn Lys Ile Tyr Val Glu Val Tyr Asp Ile Ile Ser

                    2100                                      2130                                     2160
AAT GCG ATA GTG AAG CAT AAT AAA ATT CAT AAA AAC ATA GAA TCA GTT GAT GAC AAT ACC TAC ATT TCT AAT TTG CCT TAT ACC ATC AAA
Asn Ala Ile Val Lys His Asn Lys Ile His Lys Asn Ile Glu Ser Val Asp Asp Asn Thr Tyr Ile Ser Asn Leu Pro Tyr Thr Ile Lys

                    2190                                      2220                                     2250
TAC AAA ATA TTC GAG CAA CAA TAA GTA TTT TTT ATA CCT TTA AAA TTG ATA AAT AAA TTT TTT CTA GTG ATA TTT TGG CAA GAT GAG AAT
Tyr Lys Ile Phe Glu Gln Gln End
                    2280
CCT ATT TCT CAT CGC TTT CAT GTA TGG GTG TGT TCA CTC ATA TGT TAA C
```

<div align="center">

# FIG_6

</div>